Europäisches Patentamt

European Patent Office

Office européen des brevets

(11) Veröffentlichungsnummer: **0 274 385 B1**

(12) **EUROPÄISCHE PATENTSCHRIFT**

(45) Veröffentlichungstag der Patentschrift: **15.04.92**

(21) Anmeldenummer: **88100085.5**

(22) Anmeldetag: **07.01.88**

(51) Int. Cl.⁵: **G01N 33/00**

(54) **Nachweisgerät für Gase mit einer während der Messung vom Probenraum absperrbaren Diffusionsmesskammer.**

(30) Priorität: **09.01.87 DE 3700460**

(43) Veröffentlichungstag der Anmeldung:
**13.07.88 Patentblatt 88/28**

(45) Bekanntmachung des Hinweises auf die
Patenterteilung:
**15.04.92 Patentblatt 92/16**

(84) Benannte Vertragsstaaten:
**BE DE ES FR GB NL SE**

(56) Entgegenhaltungen:
**EP-A- 0 006 256**
**DE-A- 3 541 530**
**DE-U- 7 003 943**
**US-A- 3 854 320**

(73) Patentinhaber: **Drägerwerk Aktiengesellschaft**
**Moislinger Allee 53-55**
**W-2400 Lübeck 1(DE)**

(72) Erfinder: **Matthiessen, Hans, Dipl.-Ing.**
**Heischbrook 22**
**W-2407 Gross-Parin(DE)**

**Beschreibung**

Die Erfindung betrifft ein Gerät zum Nachweis eines gasförmigen Bestandteils in Luft mit den Merkmalen des Oberbegriffs des Patentanspruchs 1.

Bei einem Nachweisgerät nach der US-A-4 384 925 ist in einer Strömungsleitung für eine zu untersuchende Luftprobe ein Sensorgehäuse mit einem Sensor angeordnet. Von Zeit zu Zeit wird die Strömungsleitung stromaufwärts und stromabwärts des Sensors unterbrochen, so daß eine definierte Menge der Luftprobe in diesem Leitungsabschnitt und dem Sensorgehäuse eingeschlossen ist. Der Sensor arbeitet nach dem coulometrischen Prinzip, so daß die gemessenen Ladungsträger während einer durch eine Steuereinheit vorgegebenen Zeitspanne ein Maß für die in dem abgeschlossenen Luftprobenraum enthaltenen Gaskonzentration sind. Dieser wert wird dann als kalibrierwert für die sich daran anschließende kontinuierliche Messung durch einen unabhängigen zweiten Sensor verwendet.

Bei dem bekannten Nachweisgerät ist jedoch der Kalibriersensor auch vor der eigentlichen Kalibration ständig der Luftprobe ausgesetzt, so daß bei Beginn der Kalibration, das heißt bei Abschließung des Sensorgehäuses, eine nicht reproduzierbare, sich oft verändernde Gaskonzentration vorherrscht. Diese unstabilen Meßbedingungen können die Meßzeit bis zur Erreichung stabiler Zustande erheblich hinauszögern. Außerdem sind die abgetrennten Abschnitte der Strömungsleitung ebenfalls als Teile der Meßkammer anzusehen, in denen sich Nester der Luftprobe von anderer Konzentration als in der Sensorkammer einfangen können, die zur Verfälschung des Meßergebnisses beitragen.

Ein weiteres Nachweisgerät ist aus der US-A-3 854 320 bekanntgeworden. Dieses besitzt einen Probenraum, in welchen das zu untersuchende Gas, in diesem Falle das Ausatemgas eines Probanden, über einen Einlaß hineingeleitet, und nach dessen Durchspülung über einen offenen Auslaß in die Umgebung abgegeben wird. Im Innern des Probenraumes befindet sich ein während der Spülphase mit einer Kapselung bedeckter Sensor zum Nachweis eines in der Luftprobe enthaltenen Gasbestandteils, in dem bekannten Falle handelt es sich dabei um Atemalkohol. Wenn nach genügend langer Spülzeit eine signifikante Luftprobe im Probenraum vorhanden ist, wird die Kapsel über dem Sensor abgehoben, so daß dieser nun frei der zu untersuchenden Luftprobe in dem Probenraum ausgesetzt ist. Während dieser Expositionszeit gibt der Sensor ein Signal an eine Auswerteeinheit ab, welche daraus die Konzentration der nachzuweisenden Gaskomponente im Probenraum angibt. Während der Meßzeit ist der Einlaß verschlossen, so daß keine weitere Luftprobe zuströmen kann. Soll der Meßvorgang beendet werden, wird die Kapsel wiederum über den Sensor geschoben, so daß dieser gegenüber dem Probenraum abgeschlossen ist. Zur gleichen Zeit wird der Sensor und der von der Sensorkapsel umschlossene Raum mit Umgebungsluft gespült, um jede Restmenge von noch vorhandenem Probengas im gekapselten Sensorraum zu entfernen.

Bei dem vorbekannten Nachweisgerät ist der Probenraum während der Messung strömungsmäßig lediglich vom Einlaß abgetrennt, jedoch findet durch den offenen Auslaß ein ständiger, nicht unerheblicher Gasaustausch mit der Umgebungsatmosphäre statt, so daß die anfängliche Konzentration des nachzuweisenden Gases während der Messung ständig durch äußere Einflüsse abnimmt.

Bei einem Nachweisgerät nach der EP-A-6256 wird die zu untersuchende Gasprobe, z.B. Atemgas, durch eine Spülkammer gefördert, bis diese nahezu vollständig mit dem Probengas angereichert ist. Während dieser Zeit ist die Meßkammer mit dem Sensor über ein Schaltventil einerseits von der Spülkammer getrennt, andererseits mit einer Frischluftleitung verbunden, durch die mit Hilfe einer Pumpe eine genau einstellbare Menge an Frischluft am Meßsensor vorbeiströmt. Nach einer bestimmten Zeitperiode werden gleichzeitig ein eingangs der Spülkammer befindliches Umschaltventil und das ausgangs der Spülkammer befindliche Schaltventil betätigt, so daß nunmehr die Spülkammer sowohl mit der Frischluftleitung als auch mit der Meßkammer verbunden ist. Das Frischgas drückt jetzt die in der Spülkammer gesammelte Luftprobe mit definierter Strömungsgeschwindigkeit in die Meßkammer und an dem Meßsensor vorbei, der auf diese Weise die Menge einer im Probengas enthaltenen, zu untersuchenden Gaskomponente bestimmt.

Auch bei diesem Nachweisgerät wird zum einen der Meßsensor während der Sammelzeit, d.h. während der Füllung der Spülkammer mit Probengas, durch die ständige Luftströmung aus der Frischluftleitung gestört, und auch während der Meßperiode, d.h. wenn das Probengas aus der Spülkammer in die Meßkammer geleitet wird, erfolgt die Messung stets während einer nicht unerheblichen Luftströmung. Bei der Austreibung des Probengases aus der Spülkammer mit Hilfe der Frischluft kommt es zu einer Verdünnung des Probengases in der Spülkammer mit der Frischluft, so daß eine zuverlässige, gleichbleibende Anzeige der nachzuweisenden Gaskomponente nicht gewährleistet ist.

Es ist daher wünschenswert, ein Nachweisgerät der genannten Art so zu verbessern, daß der Sensor vor Beginn der Meßperiode einen stabilen, vom Prüfgasstrom unbeeinflußten Ausgangszustand einnimmt, und daß der Probenraum zur Erreichung einer kurzen Meßzeit ein möglichst kleines Volumen bei gleichzeitig niedrigem Strömungswiderstand für die Luftprobe aufweist.

Die Verbesserung des Nachweisgerätes wird durch die Merkmale des kennzeichnenden Teils des Patentanspruchs 1 erreicht.

Durch die Erfindung wird erreicht, daß vor Beginn der Meßperiode dem Sensor stabile Ausgangswerte vorgegeben werden, weil er vor der Probenahme von der die Spülkammer durchströmenden Luftprobe abgedeckt ist.

Ein unter Eigenverbrauch der nachzuweisenden Gaskomponente arbeitender abgedeckter Sensor würde dabei sogar selbsttätig auf seinen Nullpunkt als Ausgangswert zurückgeführt.

Außerdem verfälscht ein abgedeckter Sensor nicht durch vorzeitigen Verbrauch von nachzuweisenden Gasbestandteilen während der Probenahme das spätere Meßergebnis.

Dem Sensor wird in seiner Meßkammer eine definierbare Menge an Probenluft aus dem Spülraum zugeführt, welche durch die Festlegung der Meßkammergröße bestimmt ist. Dabei ergibt ein geringes Meßkammervolumen eine entsprechend kurze Meßzeit. Dennoch kann ein genügend kleiner Strömungswiderstand des Spülkammervolumens beibehalten bleiben, so daß beispielsweise eine Probenahme durch Ausatmen einer Person in die Spülkammer ermöglicht ist. Die so eingefangene Probenmenge kann ohne störende äußere Einflüsse aus dem Spülraum einer quantitativen Analyse unterzogen werden. Insbesondere kann dies bei Verwendung von elektrochemischen Sensoren durch eine sehr genaue coulometrische Messung durchgeführt werden. Störungen des Meßvorganges durch eventuelle Verdünnung der Luftprobe in der Meßkammer sind ausgeschlossen.

Da die Meßkammeröffnung als eine Diffusionsstrecke ausgebildet ist, kann die in der Meßkammer herrschende Konzentration der nachzuweisenden Gaskomponente durch die Öffnungsdauer der Diffusionsstrecke beeinflußt werden, wenn diese unterhalb der Zeit für die Einstellung eines Gleichgewichtes zwischen den Konzentrationen in der Spülkammer und der Meßkammer angesetzt wird.

Für die Durchführung einer Messung bei sehr hohen Konzentrationen, die den Sensor in eine Sättigung treiben würden, kann die Konzentration auf diese Weise unter Einbeziehung experimentell bestimmbarer Korrekturfaktoren erheblich abgeschwächt werden, so daß eine analytische quantitative Messung möglich bleibt.

Für den Fall einer coulometrischen Messung wird nur eine geringe Teilmenge der in der Spülkammer zur Verfügung stehenden Luftprobe zur Messung herangezogen, die in einer erheblich kürzeren Zeit aufgebraucht ist, als es für die Gesamtmenge der Fall wäre.

Weil nach Ablauf der Zeitperiode sowohl der Einlaß als auch der Auslaß geöffnet sind, kann schon während des Meßvorganges der Spülraum entweder mit Frischluft gespült oder eine erneute Probenahme begonnen werden.

Die Spülkammer besteht aus einem Gehäuse, in welches sowohl der Einlaß als auch der Auslaß sowie die Diffusionsstrecke münden, wobei die beiden ersten Mündungen durch eine Einlaßdichtung und Auslaßdichtung auf einem gemeinsamen Dichtungsträger verschließbar sind, welcher von einem in die Spülkammer hineinragenden, von einem Stellglied axial verschiebbaren Stößel aufgenommen ist und welcher weiterhin einen Diffusionsstreckenverschluß derart angeordnet aufweist, daß die Diffusionsstrecke bei geöffnetem Einlaß und Auslaß geschlossen, und bei geschlossenem Einlaß und Auslaß geöffnet ist. Somit kann in einfacher Weise mit einem Ventilhub sowohl die Spülkammer von der sie durchströmenden Luftprobe abgeschlossen und gleichzeitig die Diffusionsstrecke zur Meßkammer geöffnet werden. Bei erneutem Verschluß der Diffusionsstrecke werden Einlaß und Auslaß zur Spülkammer wieder geöffnet.

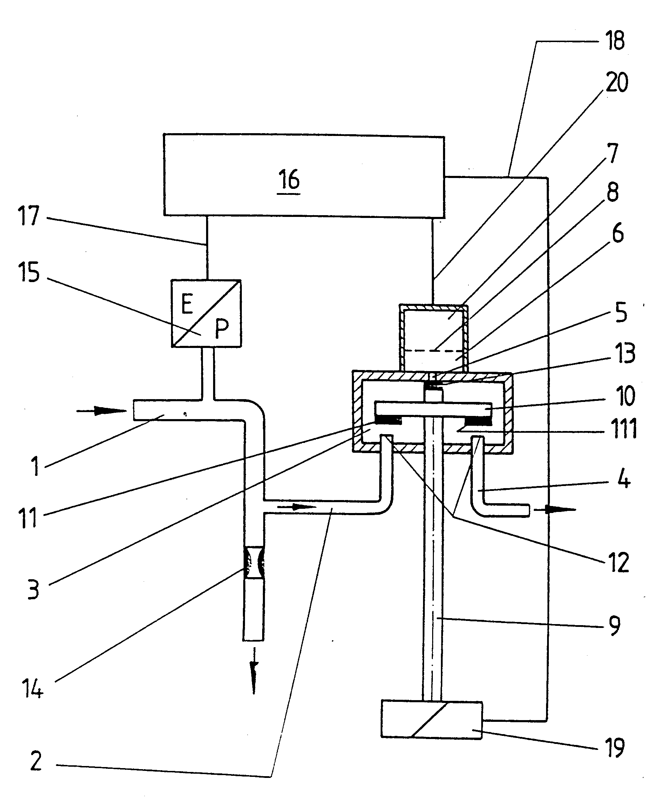

Ein Ausführungsbeispiel der Erfindung wird anhand einer schematischen Darstellung erläutert und im folgenden näher beschrieben. In der einzigen Figur ist eine Prüfluftleitung (1), durch welche die zu untersuchende Luftprobe in Richtung der dargestellten Pfeile gefördert wird, zunächst im Hauptstrom über eine Drossel (14) mit der Umgebung verbunden. Von der Prüfleitung (1) zweigt ein enger Einlaß (2) in eine Spülkammer (3, die über einen Auslaß (4) mit der Umgebung verbunden ist. Die Spülkammer (3) weist darüberhinaus eine Diffusionsstrecke (5) auf, welche die Verbindung zu einer Meßkammer (6) für einen Nachweissensor (7) herstellt. Der Sensor (7) ist von der Meßkammer (6) durch ein Trennelement (8) abgeteilt, welches für den Fall eines elektrochemischen Sensors dessen Diffusionsmembran ist. In die Spülkammer (3) ragt ein Ventilstößel (9) hinein, der an seinem Ende einen Dichtungsträger (10) aufweist. Er ist auf der Stirnfläche des Stößels (9) montiert und besitzt an seinen beiden Enden gummielastische Druckstücke (11, 111), welche auf die Mündungen (12) des Einlasses (2) und des Auslasses (4) weisen. In der Verlängerung des Stößels (9) besitzt der Dichtungsträger (10) ein Verschlußstück (13), welches die Diffusionsstrecke (5) verschließt.

Zur Durchführung einer Luftprobenmessung wird zunächst, wie in der Figur dargestellt, die Luftprobe durch die Prüfluftleitung (1) geleitet, aus welcher der größte Teil in die Umgebung abströmt. Durch die Drossel (14) wird in der Leitung (1) ein Druck aufgebaut, welcher durch einen Drucksensor (15) einer Steuereinheit (16) über eine Druckmeßleitung (17) mitgeteilt wird. Ein Teilstrom der Luftprobe wird über den geöffneten Einlaß (2) in die Spülkammer (3) geleitet, in ihr verteilt und aus dem Auslaß (4) an die Umgebung abgegeben. Sofern über eine an der Steuereinheit (16) vorgebbare Zeit ein bestimmter Druck am Drucksensor (15) geherrscht hat, wird der Ventilstößel (9) durch ein von der Steuereinheit (16) über eine Ventilsteuerleitung (18) ansteuerbares Ventil (19) in eine Position verschoben, in welcher die Mündungen (12) des Einlasses (2) und des Auslasses (4) durch die Druckstücke (11, 111) gleichzeitig verschlossen werden. Zur selben Zeit wird die Diffusionsstrecke (5) wegen des sich von ihr abhebenden Verschlußstückes (13) geöffnet und die in der Spülkammer (3) befindliche Luftprobe kann durch die Diffusionsstrekke (5) in die Meßkammer (6) eindiffundieren. Der Nachweissensor (7) ist durch die Steuereinheit (16) über die Sensorleitung (20) für eine Messung aktiviert. Nach Ablauf einer durch die Steuereinheit (16) vorgebbaren Zeitperiode wird das Ventil (19) betätigt und der Ventilstößel wieder in die dargestellte Lage zurückversetzt. Damit ist eine durch die Öffnungszeit der Diffusionsstrecke (5) bestimmbare Luftprobenmenge in die Meßkammer (6) eingedrungen, die nunmehr ungestört von irgendwelchen Einflüssen im Probenahmesystem zur Auswertung zur Verfügung steht. Bei Verwendung einer coulometrischen Messung wird das nachzuweisende Gas durch den Nachweissensor (7) verzehrt, so daß der gemessene, durch den elektrochemischen Sensor (7) fließende Strom von der Steuereinheit (16) ausgewertet und als Konzentrationsangabe angezeigt werden kann.

## Patentansprüche

1. Gerät zum Nachweis eines gasförmigen Bestandteils in Luft, welches eine einen Sensor (7) enthaltende Meßkammer (6) besitzt, die von einer Luftprobe aus einer über jeweils einen schaltbaren Einlaß (2) und Auslaß (4) abgetrennten Spülkammer (3) durchströmbar ist, wobei die Meßkammer (6) über eine von der Spülkammer (3) trennbare Meßkammeröffnung (5) verbunden ist, dadurch gekennzeichnet, daß die Meßkammeröffnung als eine Diffusionsstrecke (5) ausgebildet ist und daß zur Festlegung der in die Meßkammer (6) diffundierenden Probenmenge zu Beginn einer bestimmbaren Zeitperiode sowohl der Einlaß (2) als auch der Auslaß (4) verschlossen und gleichzeitig die Meßkammeröffnung (5) geöffnet sind und daß nach Ablauf der Zeitperiode sowohl der Einlaß (2) als auch der Auslaß (4) geöffnet sowie die Meßkammeröffnung (5) geschlossen sind.

2. Gerät nach Anspruch 1, dadurch gekennzeichnet, daß die beiden Mündungen (12) vom Einlaß (2) und Auslaß (4) durch eine Einlaßdichtung (11) und eine Auslaßdichtung (111) auf einem gemeinsamen Dichtungsträger (10) verschließbar sind, welcher von einem in die Spülkammer (3) hineinragenden, von einem Stellglied (19) axial verschiebbaren Stößel (9) aufgenommen ist, und welcher weiterhin einen Diffusionsstreckenverschluß (13) derart angeordnet aufweist, daß die Diffusionsstrecke (5) bei geöffnetem Einlaß (2) und Auslaß (4) geschlossen und bei geschlossenem Einlaß (2) und Auslaß (4) geöffnet ist.

## Claims

1. Device for the detection of a gaseous component in air, which has a measuring chamber (6) containing a sensor (7), through which measuring chamber (6) an air sample can flow from a separated rinsing chamber (3) via a controllable inlet (2) and outlet (4) respectively, whereby the measuring chamber (6) is connected by means of a measuring-chamber opening (5) separable from the rinsing chamber (3), characterized in that the measuring-chamber opening is constructed as a diffusion path (5) and in that to ascertain the sample quantity diffusing into the measuring chamber (6), at the beginning of a determinable period of time, both the inlet (2) and the outlet (4) are closed and at the same time the measuring-chamber opening (5) is opened and in that after the course of the period of time both the inlet (2) and the outlet (4) are opened as soon as the measuring-chamber opening (5) is closed.

2. Device according to claim 1, characterized in that both openings (12) from the inlet (2) and outlet (4) can be closed by an inlet seal (11) and an outlet seal (111) on a common seal support (10), which is lifted up by a push rod (9) projecting into the rinsing chamber (3) and axially displaceable by a positioning element (19), and which seal support (10) furthermore has a diffusion path closure (13) arranged in such a way that the diffusion path (5) is closed with inlet (2) and outlet (4) open and is open with inlet (2) and outlet (4) closed.

## Revendications

1. Appareil de détection d'un constituant gazeux de l'air, qui possède une chambre de mesure (6) contenant un capteur (7), laquelle peut être traversée par un échantillon d'air à partir d'une-chambre de balayage (3) séparée, par une entrée (2) et une sortie (4) commutables, la chambre de mesure (6) étant reliée par une ouverture de chambre de mesure (5) pouvant être séparée de la chambre de balayage (3), caractérisé en ce que l'ouverture de chambre de mesure est conçue sous la forme d'un parcours de diffusion (5) et en ce que pour déterminer la quantité d'échantillon diffusant dans la chambre de mesure (6), l'entrée (2) ainsi que la sortie (4) sont fermées au début d'une période de temps à déterminer et en même temps l'ouverture de chambre de mesure (5) est ouverte et en ce qu'à l'expiration de cette période de temps, l'entrée (2) ainsi que la sortie (4) sont ouvertes et l'ouverture de chambre de mesure (5) est fermée.

2. Appareil selon la revendication 1, caractérisé en ce que les deux orifices (12) de l'entrée (2) et de la sortie (4) peuvent être fermés par une garniture d'étanchéité d'entrée (11) et une garniture d'étanchéité de sortie (111) sur un support d'étanchéité (10) commun qui est logé par un poussoir (9) s'engageant dans la chambre de balayage (3), déplaçable axialement par un organe de réglage (19) et qui comporte en outre une fermeture de parcours de diffusion (13), disposée de telle sorte que le parcours de diffusion (5) soit fermé lorsque l'entrée (2) et la sortie (4) sont ouvertes et soit ouvert, lorsque l'entrée (2) et la sortie (4) sont fermées.